# EUROPEAN PATENT APPLICATION

(11) **EP 4 585 207 A1**
(43) Date of publication of application: **16.07.2025**
(21) Application number: 24223636.2
(22) Date of filing: 30.12.2024
(51) Int. Cl.: A61K 9/00, A61K 9/16, A61K 31/44, A61P 29/00

(54) **SYNERGIC AND STABLE PHARMACEUTICAL COMPOSITION OF AN NSAID AND AN OPIOID ANALGESIC FOR PAIN AND INFLAMMATION**

(30) Priority: 12.01.2024 MX 2024000705
(71) Applicant: Laboratorios Silanes, S.A. de C.V., Mexico, 11000 (MX)
(72) Inventor: González Canudas, Jorge Alejandro, 11000 Ciudad de México (MX); Espinosa Olivares, Marco Antonio, 11000 Ciudad de México (MX); Muñoz Martínez, Cecilia Jannette, 11000 Ciudad de México (MX); Ollervides Rubio, Paola Yazmín, 11000 Ciudad de México (MX); Sander Padilla, Guillermo, 11000 Ciudad de México (MX)
(74) Representative: Hoffmann Eitle

(57) **Abstract**

The present invention relates to pharmaceutical compositions comprising etoricoxib and tramadol, or a pharmaceutically acceptable salt thereof, for the treatment of pain, which in addition to offering the possibility of an analgesic effect with a decrease in adverse events due to the reduction of the dose of one or both compounds, presents an improved stability, maintaining the dissolution and bioavailability of the composition to be administered; The composition manages to overcome the technological complexity that lies in having a drug with high sensitivity to light and humidity (tramadol), together with another fat-soluble drug with poor flow and excessive adhesiveness (etoricoxib), in a dispensing media in which their absorption is not affected through a water-free process, without affecting the release of both drugs.

## Description

### FIELD OF THE INVENTION

The present invention relates to combined pharmaceutical compositions, synergy and manufacturing methods thereof in the field of treatment, pain control and inflammation.

### BACKGROUND OF THE INVENTION

Pain management requires the generation of analgesic strategies that guarantee an earlier, more effective and safer relief that limits its progression towards chronic pain. Pain treatment should focus on immediate actions that prevent the development of future complications associated with disability, with the objective of improving the patient's quality of life.

Given the complex pathophysiology of pain, multimodal analgesia has been implemented as a therapeutic strategy, defined as the simultaneous use of drugs with different complementary mechanisms of action that generate a synergistic effect. Several drugs are commonly used in multimodal analgesia, including opioids, NSAIDs, and paracetamol.

According to the International Association for Pain Studies (IASP), neuropathic pain is the pain that arises as a direct consequence of an injury or disease that affect the somatosensory system.

It is known that patients with acute neuropathic pain sometimes do not respond to monotherapies, even at maximum doses. Reason for which the treatment usually involves the use of two or more drugs with complementary mechanisms of action.

For example, tramadol of formula I, whose nomenclature is (1R,2R)-2-[(dimethylamino)methyl]-1-(3-methoxyphenyl) cyclohexan-1-ol, is a synthetic codeine analogue and central action analgesic that possesses opioid agonist properties, activates the monoaminergic spinal pain inhibition, and it is commonly used for the treatment of acute pain of neuropathic origin.

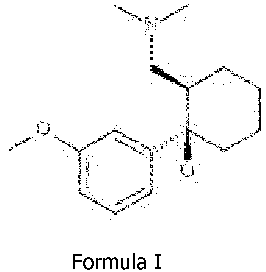

It can be administered orally, rectally, intravenously or intramuscularly. In patients with moderate to severe post-operatory pain, intravenous or intramuscular tramadol has generally been shown to be equivalent in potency to pethidine (meperidine) and one-fifth the potency of nalbuphine. Tramadol has demonstrated analgesic activity in various animal models and in healthy volunteers with experimentally induced pain. The analgesia of tramadol dosed orally is similar to that obtained intravenously (Lee, Rhoda; McTavish, Donna; Sorkin, Eugene; tramadol; A Preliminary Review of its Pharmacodynamic and Pharmacokinetic Properties, and Therapeutic Potential in Acute and Chronic Pain States, 1993)**.**

Adverse reactions commonly associated with the administration of tramadol may include constipation, nausea, vomiting, abdominal pain, dizziness, drowsiness, fatigue, and headache. Some of its adverse reactions can be serious, including: seizures, hives, blisters, difficulty swallowing, inflammation of the eyes, face, throat, tongue, lips, hands, feet, ankles; and even changes in heart rate, however, the incidence of serious adverse reactions is less than 1%. Likewise, tramadol is within group IV of opioid medications: opioid with medicinal use and low addictive potential. It is known that high doses of tramadol can cause greater adverse reactions. Intentional and accidental overdoses of tramadol can cause respiratory arrest, as well as acute liver failure, several fatal cases have been reported. In these cases, however, liver injury may have been caused by shock, hypoxia, or ischemia secondary to respiratory arrest. Liver injury attributed to tramadol overdose has also been associated with hyperammonemia, lactic acidosis, and hepatic steatosis, which suggest direct mitochondrial injury (Liver Tox Clinical and Research information of Drug Induce Livered Injury, National Institutes of Health, December 05, 2012)**.**

On the other hand, there is etoricoxib, also known as 5-chloro-2-(6-methylpyridin-3-yl)-3-(4-methylsulfonylphenyl) pyridine, of formula II. It is a synthetic non-steroidal anti-inflammatory drug (NSAID) with potential antipyretic, analgesic and antineoplastic properties. Etoricoxib specifically binds to and inhibits the enzyme cyclooxygenase-2 (COX-2), resulting in the inhibition of the conversion of arachidonic acid in prostaglandins. (https://pubchem.ncbi.nlm.nih.gov/compound/123619).

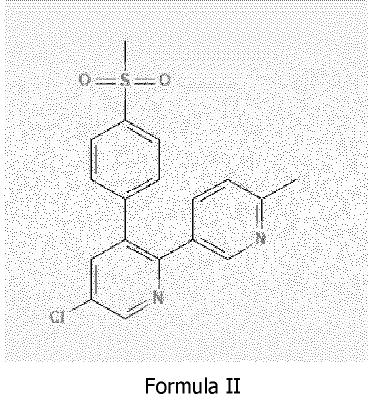

Etoricoxib is indicated for the treatment of chronic and acute pain, which is defined according to the International Association for Pain Studies (IASP) as pain that persists for more than 3 to 6 months from the moment of tissue damage. Large doses of etoricoxib can cause adverse reactions such as ulceration and intolerance in the gastrointestinal tract, blockage of platelet aggregation (inhibition of thromboxane synthesis), inhibition of uterine motility (prolongation of gestation), inhibition of renal function mediated by prostaglandins, hypersensitivity, among others.

In the state of the art, drug combinations have been developed that involve the combination of active ingredients such as etoricoxib/tramadol as an alternative for the treatment of pain and inflammation. For example, the patent application with international publication number WO 2019/130049 provides a pharmaceutical combination of prolonged release tramadol hydrochloride, and immediate release etoricoxib in combination with solvents and vehicles, its manufacturing process in a single dosage form such as capsules, tablets, bilayer tablets, granules and sachets. Additionally, it provides methods to prevent and treat pain, such as acute pain. In one embodiment, the pharmaceutical combination comprises a first formulation of tramadol and a second formulation of etoricoxib that are in granule form.

Subsequently, the Mexican application MX/a/2016/006464 describes a pharmaceutical composition of a combination of tramadol hydrochloride / etoricoxib of a specific particle size (d90 < 30 microns of etoricoxib) for the treatment of pain, as well as its manufacturing process for a mixture of powders, or tablets in a bilayer tablet with or without coating or in a capsule as granules and/or tablets with or without coating. The concentration of tramadol/etoricoxib is 10-120 mg of etoricoxib and 10-300 mg of tramadol.

The patent application with international publication number WO 2019 021079 describes a process and pharmaceutical composition of a combination of tramadol hydrochloride / etoricoxib for the treatment of pain, the process to obtain it which is given from two active ingredients which consists of granules and /or multiparticulates with tramadol hydrochloride and granules and/or multiparticulates with etoricoxib which may be contained in a mixture of powders, or tablets in a bilayer tablet with or without coating or in a capsule as granules and/or tablets with or without coating of etoricoxib 0.15 to 0.85 with regards to the total etoricoxib administered plus tramadol hydrochloride or similar salts. The concentration of tramadol / etoricoxib is from 10-300 mg to 10-120 mg, respectively. The concentration of tramadol / etoricoxib is from 10-300 mg to 10-120 mg, respectively.

Other examples of combinations include patent number MX 243332, which describes the use of a combination of an opioid analgesic together with a COX-2 inhibitor. The opioid analgesic and COX-2 inhibitor may be administered orally, by implant, parenterally, sublingually, rectally, topically, or by inhalation. In other embodiments of the invention, the COX-2 inhibitor may be administered separately from the opioid analgesic. Pharmaceutical compositions containing the COX-2 inhibitors and opioid drugs described in MX 243332 are administered orally. Such oral dosage forms may contain one or both drugs in immediate or extended release form. For ease of administration, it is preferred that the oral dosage form contains both drugs. Oral dosage forms may be in the form of tablets, pieces, lozenges, aqueous or oily suspensions, dispersible powders or granules, emulsions, multiparticulate formulations, syrups, elixirs and the like.

Likewise, the publication of patent application MX/a/2019/008577 describes the combination of etoricoxib / tramadol for the treatment of pain through an independent dual-release single-dose system that comprises a COX-2 inhibitor and a central action opioid, which is made up of two matrices and is useful for the treatment of moderate to severe pain. The process for obtaining said independent release matrix is also described.

For its part, US patent document US 2008/0026054 describes a slow-release pharmaceutical composition containing tramadol and an immediate-release layer of an NSAID that includes, but is not limited to, etoricoxib in tablet or capsule pharmaceutical form.

Tramadol / etoricoxib co-crystals have even been described in patent document MX 311821 for the preparation of a medication indicated for the treatment of pain.

On the other hand, the publication called "Analysis of interaction between etoricoxib and tramadol against mechanical hyperalgesia of spinal cord injury in rats." (Pal Singh, Vijay; Patil, Chandrashekhar S.; Kulkarni, Shrinivas K.; Life Sciences (2006), 78(11), 1168-1174.) presents a non-clinical or pre-clinical study to evaluate the pharmacodynamic effects and any possible interactions (additive or potentiation) in the anti-nociceptive effects of etoricoxib; a cyclooxygenase-2 inhibitor and tramadol; a typical opioid agonist to test spinal cord injury-induced hyperalgesia in rats.

In the prior art, an attempt has been made to solve the problem of combining both drugs for the treatment and control of pain and inflammation. The documents talk a lot about bilayers and independent systems. There were even those who approached the problem from a new crystalline form. The only way they approach combining tramadol and etoricoxib is that there is no actual physical interaction of the APIS, otherwise they are not combined.

Likewise, there is a need to improve the synergistic effect, increase bioavailability, improve dissolution, stability and at the same time ensure a decrease in side effects once administered to the patient. The lack of solutions and alternatives to the above causes the therapeutic effect to take time to occur or a higher dose to be required. Furthermore, the patents and scientific contributions described above do not show interest in resolving the impact on the organoleptic properties of the drug, which affect its stability, dissolution and bioavailability.

On the other hand, in addition to the problems of side effects due to the conformation of high doses of tramadol and etoricoxib, there is the technical problem of combining a drug with high sensitivity to light and humidity, with another fat-soluble drug with poor flow and excessive adhesiveness since there could be interference in the stability, absorption and release mechanism.

Thus, the present invention refers to a pharmaceutical composition of a combination of tramadol - etoricoxib, a single monophasic system manufactured by fusion granulation, stable, immediate release, with a synergistic effect and which maintains dissolution and bioavailability, in the field of treatment and control of pain and inflammation.

### BACKGROUND OF THE INVENTION

The present invention relates to granulated and reconstitution pharmaceutical compositions comprising tramadol and etoricoxib, or a pharmaceutically acceptable salt thereof. The compositions of the present invention are used in the manufacture of a medicament useful in the treatment of pain and inflammation, which in addition to offering the possibility of an analgesic effect with a decrease in adverse events because the active ingredients are in an effective amount to provide a synergistic pharmacological effect, reducing the dose of one or both compounds, presents improved stability, maintaining the dissolution and bioavailability of the composition to be administered.

The enhanced analgesic effect of the present invention is due to the combination of two pharmacodynamic mechanisms that complement each other thanks to the combination of an NSAID (etoricoxib) plus an opioid (tramadol). Additionally, this combination represents a safety profile superior to the treatment with monodrugs since it allows the adverse events derived from the use of opioids to be reduced because when combining etoricoxib with tramadol, therapeutic results are obtained with a daily dose of the product. On the one hand, the pharmacokinetic profile of the etoricoxib/tramadol combination of the present invention demonstrates that the reduction in the dose of tramadol (tramadol-sparing effect) is not related to pharmacokinetic differences of the fixed-dose formulation of etoricoxib/tramadol, but rather to a synergistic effect based on pharmacodynamics inherent to the mechanisms of action of the drugs when administered in combination and provides a multimodal therapeutic approach. On the other hand, the treatment based on Etoricoxib/Tramadol is more powerful from a pharmacological point of view since with lower doses (90 mg/50 mg vs 975 mg/112.5 mg) the desired analgesic therapeutic effects are achieved. The results obtained through a clinical study by the applicant demonstrate analgesic synergy in patients affected by acute lower back pain, in addition to a tramadol-sparing effect. Also, among all the adverse events reported in the clinical study carried out by the inventors, the Etoricoxib/Tramadol group reported a lower proportion of AEs compared to the Paracetamol/Tramadol group.

Furthermore, an improved stability is presented regarding the pharmaceutical profile.

One embodiment of the invention comprises a synergistic composition in a single, stable, immediate release, solid dosage form, comprising tramadol hydrochloride at a concentration of 50 mg/5 g and etoricoxib 90 mg/5 g, respectively.

In another embodiment of the invention the composition is found in the form of granules. Preferably, it is in the form of a granule for reconstitution.

Also, the excipients that make up the composition providing it with essential characteristics that improve the pharmacokinetic profile, bioavailability, stability and dissolution are an object of the present invention.

Another embodiment of the invention comprises the manufacturing process of the composition which does not require the incorporation of water and manages to overcome the technological complexity that lies in having a drug with high sensitivity to light and humidity (tramadol), together with another fat-soluble drug with poor flow and excessive adhesiveness (etoricoxib), in a dispensing media in which the absorption thereof is not affected, through a fusion granulation process, reason for which, the release of both drugs is not affected.

The manufacturing process of the pharmaceutical composition according to the present invention comprises the following steps:
1) mixing the active ingredients tramadol and etoricoxib with previously screened pharmaceutically acceptable excipients for approximately 5 minutes;
2) granulate the mixture obtained in the previous stage for 20 to 30 minutes until reaching a temperature between 54 °C to 55.2 °C;
3) cool the granules from the previous step to a temperature of 25 °C to 28 °C;
4) screen the granules obtained in the previous step;
5) mix the resultant screen obtained in the previous step with a flavoring for 5 minutes.

The invention also relates to a granulated pharmaceutical composition for reconstitution comprising tramadol and etoricoxib in an effective synergistic amount of 50/90 mg respectively obtained by the fusion granulation process described; as well as its use in the manufacture of a medicament useful in the treatment and control of pain such as neuropathic pain and/or chronic and acute pain.

### BRIEF DESCRIPTION OF THE DRAWINGS

**Figure 1****.** Result of dissolution. Dissolution profiles of tramadol HCl/etoricoxib batch DFF1712-12.
**Figure 2****.** Comparison of dissolution profiles of etoricoxib batch NO11650 & DFF1908-03.
**Figure 3****.** Comparison of dissolution profiles of tramadol batch 170111 & DFF1908-03.
**Figure 4****.** Comparison of dissolution profiles of etoricoxib batch NO11650 & DFF1908-03 (B).
**Figure 5****.** Comparison of dissolution profiles of tramadol batch 170111 & DFF1908-03 (B).
**Figure 6****.** Comparison of dissolution profiles of etoricoxib batch NO11650 & DFF1908-03 (B2C).
**Figure 7****.** Comparison of dissolution profiles of tramadol batch 170111 & DFF1908-03 (B2C).
**Figure 8****.** Mean pharmacokinetic profile of etoricoxib ± standard error on normal scale. (A. reference drug (etoricoxib), C: test drug (etoricoxib - tramadol).
**Figure 9****.** Mean pharmacokinetic profile of etoricoxib ± standard error on a semi-logarithmic scale. (A. reference drug (etoricoxib), C: test drug (etoricoxib - tramadol).
**Figure 10****.** Mean pharmacokinetic profile of tramadol ± standard error on normal scale. (B. reference drug (tramadol), C: test drug (etoricoxib - tramadol).
**Figure 11****.** Mean pharmacokinetic profile of tramadol ± standard error on a semi-logarithmic scale. (B. reference drug (tramadol), C: test drug (etoricoxib - tramadol).
**Figure 12****.** Comparative pharmacokinetic profiles of etoricoxib (normal scale) (A). reference drug (etoricoxib), (C): test drug (etoricoxib - tramadol).
**Figure 13****.** Comparative pharmacokinetic profiles of etoricoxib (semi-logarithmic scale) **(A).** reference drug (etoricoxib), **(C):** test drug (etoricoxib - tramadol).
**Figure 14****.** Comparative pharmacokinetic profiles of tramadol (normal scale) **(B).** reference drug (etoricoxib), **(C):** test drug (etoricoxib - tramadol).
**Figure 15****.** Comparative pharmacokinetic profiles of tramadol (semi-logarithmic scale) **(B).** reference drug (tramadol), **(C):** test drug (etoricoxib - tramadol).
**Figure 16****.** Dissolution profiles of granules of single-drug etoricoxib, single-drug tramadol HCl, tramadol HCl batch DFF1908-03 and etoricoxib batch DFF1908-03 from Laboratorios Silanes S.A. of C.V. in 0.1N hydrochloric acid dissolution media.
**Figure 17****.** Dissolution profiles of reference drug Tradol [50 mg tramadol hydrochloride] and Arcoxia 90 mg etoricoxib, etoricoxib, tramadol HCl batch DFF1908-03 and etoricoxib batch DFF1908-03 from Laboratorios Silanes S.A. of C.V. in 0.1N hydrochloric acid dissolution media.

### DETAILED DESCRIPTION OF THE INVENTION

### Definitions

Pharmaceutically acceptable salt: The term "pharmaceutically acceptable salt" of a given compound refers to salts which retain the biological efficacy and properties of the given compound, and that are not biologically, or otherwise, undesirable (P. Heinrich Stahl and Camille G. Wermuth (Eds.) Pharmaceutical Salts Properties, Selection, and Use (International Union of Pure and Applied Chemistry), Wiley-VCH; 2d Revised Edition (May 16, 2011)).

Neuropathic pain: according to the International Association for the Study of Pain, neuropathic pain is pain that is a direct consequence of an injury or disease of the somatosensory system. And this type of pain is present in various neuropathies, polyneuropathies, post-therapeutic neuralgia, other common central pain syndromes, for example, spinal cord injury, spinal cord tumor, syringomyelia, and cancer associated with neuropathic pain.

Excipient: is the ingredient that is part of the preset pharmaceutical composition, among them are binders, disintegrants, sweeteners, flavorings, among others.

Synergy: a combined technical effect. The most favorable case is when the combination of active ingredients produces a synergistic effect, that is, two characteristics interact synergistically with each other if their functions are linked and if they give rise to an additional effect that goes beyond the sum of the effects produced for each of these characteristics considered in isolation, in this case, the individual active ingredients.

The present invention relates to a pharmaceutical composition of a combination of an opioid analgesic and a non-steroidal anti-inflammatory in a single monophasic system that is a granule to reconstitute, stable, of immediate release, with a synergistic effect and which maintains the dissolution and bioavailability. in the field of treatment and control of pain and inflammation.

The opioid analgesic used is tramadol or a pharmaceutically acceptable salt thereof, for example, the hydrochloride salt thereof and the NSAID is etoricoxib or a pharmaceutically acceptable salt thereof.

The present invention comprises the combination of etoricoxib and tramadol or a pharmaceutically acceptable salt thereof in doses of 50 mg of tramadol and 90 mg of etoricoxib; with at least one pharmaceutically acceptable excipient.

As indicated above, the solid pharmaceutical composition is in the form of a granule for reconstitution. The most preferred pharmaceutical form of the invention is in "granules to reconstitute" because the dissolution results do not show problems for tramadol hydrochloride or etoricoxib, releasing 100% of the active ingredients, in addition to being a pharmaceutical form that is better accepted by its easy swallowing.

The combination of tramadol and etoricoxib represents a set of important technological challenges due to the physicochemical properties of the drugs, the appropriate selection of excipients and manufacturing conditions play, in the development of pharmaceutical compositions, a very important role in relation to the release of the drug and the rate of absorption in the body. And so, the composition manages to overcome the technological complexity that lies in having a drug with high sensitivity to light and humidity (tramadol), together with another fat-soluble drug with poor flow and excessive adhesiveness (etoricoxib), in a dispensing media in which the absorption thereof is not affected, through a fusion granulation process, reason for which, the release of both drugs is not affected.

With regards to the excipients, in one embodiment, the pharmaceutical composition of the present invention comprises at least one of a binder, a disintegrant, a sweetener, a flavoring and a diluent.

Examples of pharmaceutically acceptable disintegrants include, but are not limited to, croscarmellose, cellulose derivatives such as hydroxypropyl cellulose, carboxymethyl cellulose, microcrystalline cellulose; povidone derivatives such as crospovidone; starch derivatives such as pregelatinized starch, sodium starch glycolate and corn starch. The disintegrant may be present in an amount ranging from 0.5% to 15% by weight, preferably between 0.5% to 5% by weight. The preferred disintegrant for the present formulation is croscarmellose sodium.

Examples of pharmaceutically acceptable binders include, but are not limited to, polyethylene glycol 6000, polyoxyethylene alkyl ethers, polyethylene oxide; polyoxyethylenesorbitan fatty acid esters; polyoxyethylene stearates and suppository bases. In a preferred embodiment, the binder is polyethylene glycol 6000, which may be present in an amount of about 43% by weight in the formulation.

In a preferred embodiment, the diluent is isomalt 801, which may be present in an amount of about 36% by weight.

Examples of pharmaceutically acceptable sweeteners include, but are not limited to, acesulfame potassium, sucralose, alitame, aspartame, isomalt, lactylol, maltitol, mannitol, neotame, ammonium saccharin, calcium saccharin, sodium saccharin, sodium cyclamate, sorbitol, sucralose, tagatose, thaumatin and xylitol. Preferred sweeteners for the present formulation are acesulfame potassium and sucralose.

Acesulfame potassium is present in an amount of approximately 3.0% by weight, while sucralose is used at 2.0% by weight in the formulation.

Pharmaceutically acceptable flavoring agents include, but are not limited to, mint-peppermint flavor, d-menthol; l-menthol; or thymol. The flavoring is present in a use range of 7 - 9% LD₅₀ (rat, oral): 3.18 g/kg, preferably used in the formulation at about 8% by weight. The preferred flavoring for the present formulation is mint-peppermint flavor.

In a more preferred embodiment, the composition comprises a diluent, a binder and two sweeteners, wherein the diluent is isomalt 801, the binder is polyethylene glycol, the first sweetener is acesulfame potassium and the second sweetener is sucralose. Regarding the remaining of the excipients, the disintegrant is croscarmellose sodium and the flavoring is mint-peppermint flavor.

On the other hand, the combination of both drugs tramadol and etoricoxib suggests a synergistic antihyperalgesic effect in animal models (Pal Singh, Vijay; Patil, Chandrashekhar S.; Kulkarni, Shrinivas K.; Analysis of interaction between etoricoxib and tramadol against mechanical hyperalgesia of spinal cord injury in rats. Life Sciences (2006), 78(11), 1168-1174.).

In another embodiment of the invention, it was found that the pharmaceutical composition does not present pharmacokinetic interaction when the combined formulation of tramadol with etoricoxib is administered.

Accordingly, in another aspect, the present invention provides a method of treating pain and inflammation, which comprises administering a composition as described above to a patient in need thereof, preferably every 24 hours.

In a preferred embodiment, the type of pain that can be treated with the present invention consists of nociceptive, neuropathic or mixed pain. Additionally, according to the duration of the clinical condition, chronic pain and acute pain are subject to treatment with it.

### Manufacturing and assessment of tests carried out to obtain an optimal formula

In the prior art, even when the combination of tramadol - etoricoxib is taught, manufacturing processes, pharmaceutical form and dosage form different from that of the present invention are described. The prior art describes bilayers and independent systems of the tramadol - etoricoxib combination. There were even those who approached the problem from a new crystalline form. The only way they approach combination is that there is no real physical interaction of the APIs, otherwise they do not combine them, and therefore there is a need for a monophasic combination of tramadol - etoricoxib in a single dosage form. This is how this application describes the manufacturing process of a single monophasic system of the tramadol - etoricoxib combination that also gives results of stability and synergy.

The manufacturing process of the present combination pharmaceutical composition of tramadol - etoricoxib proposed by the inventors, refers more particularly to a fusion granulation in a single monophasic, stable, immediate release system, with a synergistic effect and which maintains the dissolution and bioavailability, in the field of treatment and control of pain and inflammation.

### Example 1. Formula of a composition containing tramadol-etoricoxib

Next, the qualitative-quantitative formula of a granulated composition for reconstitution comprising the two active ingredients tramadol hydrochloride and etoricoxib, or any of the pharmaceutically acceptable salts thereof, is described.

**Table 1. Quantitative formula**

| % | mg/5 g. | Components | Function |
|---|---|---|---|
| 1.0 | 50.0 | Tramadol hydrochloride | Drug 1 |
| 1.8 | 90.0 | etoricoxib | Drug 2 |
| 36.0 | 1800.0 | isomalt 801 | Diluent |
| 43.20 | 2160.0 | polyethylene glycol 6000 | Binder |
| 5.0 | 250.0 | croscarmellose sodium | Disintegrant |
| 3.0 | 150.0 | acesulfame potassium* | Sweetener |
| 2.0 | 100.0 | sucralose | Sweetener |
| 8.0 | 400.0 | mint-peppermint flavor | Flavoring |

| | | | |
|---|---|---|---|
| *Equivalent to acesulfame potassium. | | | |

### Example 2. Manufacturing process of the fusion granulated composition for reconstitution

The product combined the drugs tramadol (hydrochloride) and etoricoxib in a single dosage unit, with doses of 50 mg/ 90 mg respectively, which represents a set of important technological challenges due to the physicochemical properties and the difference in doses to guarantee obtaining a stable product.

In the patents of the closest prior art, wet granulation processes that incorporate water are carried out. Such is the case, in the present invention, that if it is desired to incorporate water in the process of combining the active ingredients tramadol (hydrochloride) and etoricoxib, the result of the combination is negatively affected. Below are tests carried out for the combination of tramadol (hydrochloride) and etoricoxib of the present invention via wet method, which does not allow obtaining a stable and quality combination, while the fusion granulation represents that unitary operation that allows carrying out the invention.

### Formula assessment for extrusion-spheronization tests, incorporating ethylcellulose as a spheronization promoter.

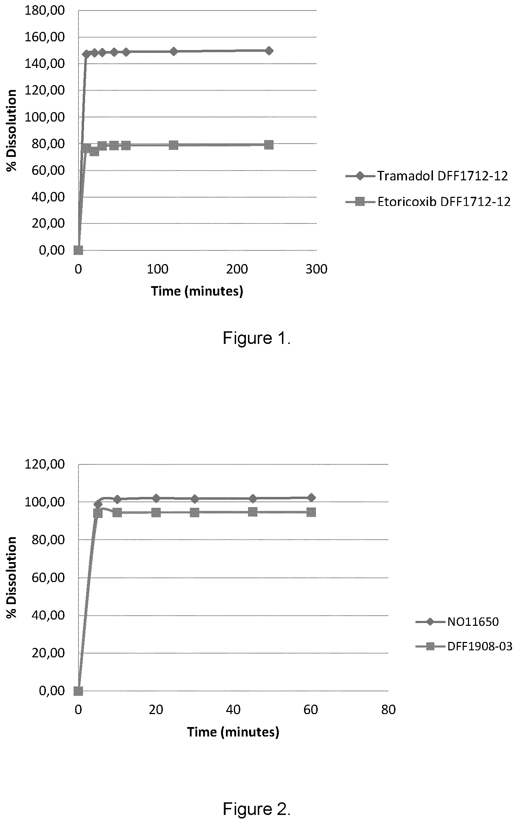

Test: 1712-12 (Figure 1).

| % | Materials |
|---|---|
| 19.94 | tramadol hydrochloride |
| 19.94 | etoricoxib |
| 15.95 | microcrystalline cellulose PH 101 |
| 44.17 | ethylcellulose E-7-7050 |

### Objective:

Formula assessment for extrusion-spheronization tests, incorporating ethylcellulose as a spheronization promoter.

### Physical results:

Disintegration 8 h 30 minutes, total weight with capsule 458.45 mg.

### Observations:

Tramadol hydrochloride and etoricoxib spheres were encapsulated; capsule weight 251 mg for tramadol HCl and 112.45 mg of etoricoxib in capsule number zero, are sent for analysis.

According to the dissolution results, problems were found for both tramadol HCl for which the release was above 100% and for etoricoxib was below, approximately 80%, this behavior is attributable to the poor distribution of the active ingredients in the formulation of the spheres. A proposal was made to change the pharmaceutical form; from extrusion-spheronization to granulation to reconstitute through wet granulation.

### Manufacturing of batch for assessment of organoleptic properties in formula.

### Test: 1810-29

| % | Materials |
|---|---|
| 1.00 | tramadol, HCl |
| 1.80 | etoricoxib |
| 40.00 | isomalt 801 |
| 52.20 | polyethylene glycol 6000 |
| 3.0 | acesulfame potassium |
| 2.0 | sucralose purified water (mL) |

### Objective:

Manufacturing of batch for formula assessment

### Observations:

A wet granulation was carried out to integrate the active ingredients into the granules. For this type of pharmaceutical form, it is important to incorporate a flavor; since the active ingredients provide a bitter taste, so it is necessary to incorporate a flavoring to improve the organoleptic properties of the active ingredients. Different flavors were tested evaluating organoleptic properties. According to the results of organoleptic properties: the bubblegum orange, grape, mint and strawberry flavors did not help to mask the bitter taste of the active ingredients, which is why it was proposed to use another flavor (Table 2).

**Table 2. Organoleptic properties**

| Experiment | 1 | 2 | 3 | 4 |
|---|---|---|---|---|
| Flavor/Descri ption | Bubblegum Orange / Liquid | Grape / Liquid | Fresh Mint / Liquid | Fresh Mint / powder |
| Concentration (%) | 0.22 | 1.0 | 0.3 | 0.3 |
| Flavor | Slightly orange / does not taste like bubblequm | Grape | Mint | Mint |
| Odor | Odorless | Grape | Mint | Mint |
| Aftertaste | Very bitter | Bitter | Slightly bitter | Slightly bitter |
| Observations | Too bitter, it does not taste like bubblegum and from the beginning it is very bitter | At first it doesn't taste bitter; the aftertaste is bitter | Takes time to dissolve, more than 2 minutes | Takes time to dissolve, less than liquid |

### Formula assessment by wet granulation

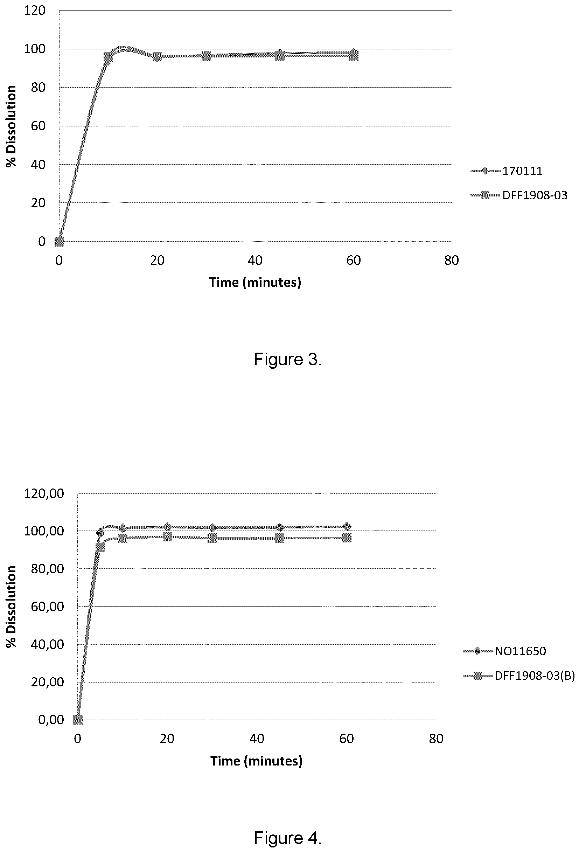

### Test: 1810-29 (Figures 2 and 3).

| % | Materials |
|---|---|
| 1.00 | etoricoxib |
| 1.80 | tramadol HCl |
| 36.0 | isomalt 801 |
| 45.7 | polyethylene glycol 6000 |
| 2.50 | croscarmellose sodium |
| 3.00 | acesulfame potassium |
| 2.00 | sucralose |
| 8.00 | mint-peppermint |
| 0.35 | purified water (mL) |

### Objective

Formula assessment by wet granulation
Physical tests
%H.R.=-0.93
Disintegration 5 minutes.
Similarity factor
etoricoxib F2= 57.51% (Reference, innovative product)
Tramadol HCl F2= 88.05% (Reference, innovative product)

### Result of dissolution

Dissolution media: buffering solution pH 2.0: Chromatographic grade acetonitrile (75:25); dissolution media: HCl.

### Observations:

In this test, the Peppermint flavor was added and 2.5% croscarmellose sodium was added. The samples were sent for analysis.

It was proposed to change to fusion granulation since the amount of water needed is minimal and a solid binder with a low melting point, between 55 °C to 62 °C, was used as an excipient.

According to the dissolution results, no problems were found for both tramadol HCl and etoricoxib, releasing 100% of the active ingredients, as an innovative batch reference for each active ingredient.

The samples obtained were subjected to a preliminary stability assessment, finding incompatibility of tramadol in the presence of residual moisture resulting from wet granulation, a color change occurs in the tablet and there is degradation of tramadol, so this manufacturing route is not viable to ensure compliance with quality attributes.

### Formula assessment by fusion granulation

On the contrary, during the fusion granulation, the physical binding of the components tramadol - etoricoxib, at least one binding agent is incorporated which melts between 50 - 80 °C, this allows the inclusion of the active ingredients without the need for the incorporation of water and eliminates the drying stage, which positively favors the combination of the active ingredients of the present invention.

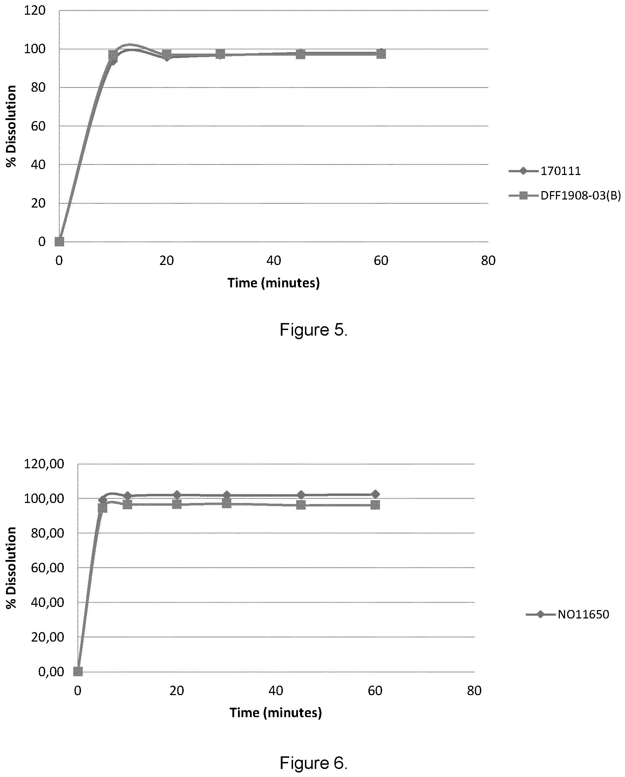

### Test: 1908-03 (B) (Figures 4 and 5).

| % | Materials |
|---|---|
| 1.00 | etoricoxib |
| 1.80 | tramadol HCl |
| 36.0 | isomalt 801 |
| 43.2 | polyethylene glycol 6000 |
| 2.50 | croscarmellose sodium |
| 3.00 | acesulfame potassium |
| 2.00 | sucralose |
| 8.00 | mint-peppermint |

### Objective

Formula assessment by fusion granulation

### Physical tests

No physical tests were carried out

### Similarity factor

etoricoxib F2= 60.51% (Reference, innovative product)

Tramadol HCl F2= 86.43% (Reference, innovative product)

### Dissolution results

Dissolution media: buffering solution pH 2.0: Chromatographic grade acetonitrile (75:25); dissolution media: HCl

### Observations

The fusion temperature range between 54.4 °C and 57.5 °C was determined for the excipient Polyethylene Glycol 6000. It was proposed to do this by doubling the amount of disintegrant up to 5%.

The dissolution results did not show problems for both tramadol HCl and etoricoxib, releasing 100% of the active ingredients, as an innovative batch reference for each active ingredient.

### Formula assessment by fusion granulation

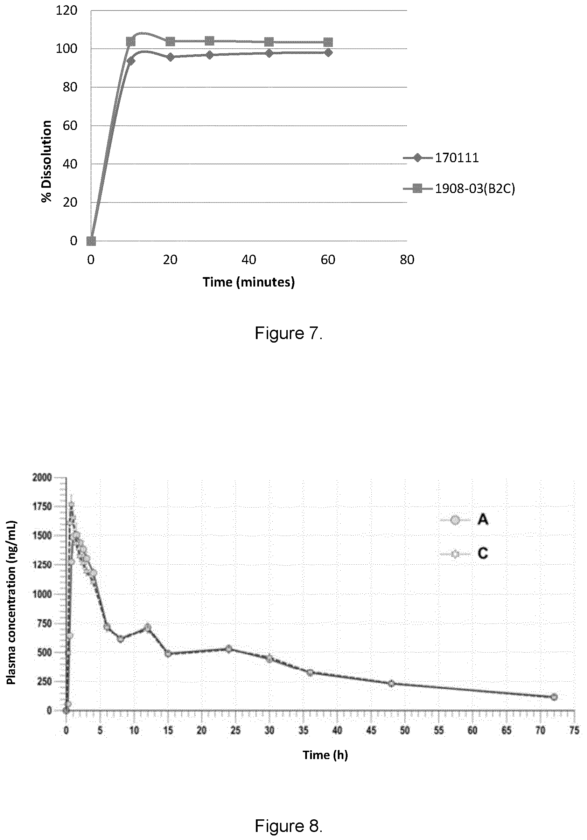

### Test: 1908-03 (B2C) (Figures 6 and 7).

| % | Materials |
|---|---|
| 1.00 | etoricoxib |
| 1.80 | tramadol HCl |
| 36.0 | isomalt 801 |
| 43.2 | polyethylene glycol 6000 |
| 2.50 | croscarmellose sodium |
| 3.00 | acesulfame potassium |
| 2.00 | sucralose |
| 8.00 | mint-peppermint |

### Physical tests

I.H.=1.09 I.C.= 8.5, angle of inertia 8.0, flow rate 10.26 g/s, average particle size 786.7

### Similarity factor

etoricoxib F2= 63.06% (Reference, innovative product)

Tramadol HCl F2= 56.12% (Reference, innovative product)

### Dissolution results

Dissolution media: buffering solution pH 2.0: Chromatographic grade acetonitrile (75:25); dissolution media: HCl.

### Observations

The formula met the similarity factors for each drug and there is reproducibility in the manufacturing process.

The similarity factor was determined with the monodrugs prepared in the same pharmaceutical form and with the innovative drugs for each active ingredient.

These results serve as a basis for preparing stability batches since an optimal formula is obtained.

According to the tests previously presented, surprisingly it was possible to develop a process that guarantees a stable product that meets the established quality characteristics, for which a fusion granulation process was selected, placing the specific excipients and additives previously mentioned, capable of maintaining the chemical integrity of both drugs together.

Below, the manufacturing process by fusion granulation of the granules to reconstitute of the present invention, comprising etoricoxib and tramadol and/or their pharmaceutically acceptable salts, is presented in a non-limiting manner:
1. Screen the Polyethylene Glycol 6000 (Screen 1).
2. Screen the etoricoxib, tramadol hydrochloride, Isomalt 801, Croscarmellose sodium, Acesulfame potassium and Sucralose (Screen 2).
3. Mix Screen 1 with Screen 2 for 5 minutes (Mixture 1).
4. Granulate Mixture 1 for 20-30 minutes until it reaches a temperature between 54-55.2 °C.
5. Cool the granules to a temperature of 25 to 28 °C.
6. Screen the granules from step 5 (Screen 3).
7. Mix Screen 3 with the meta-peppermint flavor for 5 minutes (Mixture 2).

During the fusion granulation, the physical binding of the components tramadol - etoricoxib, at least one binding agent is incorporated which melts between 50 - 80 °C, this allows the inclusion of the active ingredients without the need for the incorporation of water and eliminates the drying stage. On the contrary, the patents of the closest prior art carry out processes, for example, in which the wet granulation does incorporate water.

The product combines the drugs tramadol (hydrochloride) and etoricoxib in a single dosage unit, with doses of 50 mg/ 90 mg respectively, which represents a set of important technological challenges due to the physicochemical properties and the difference in doses to guarantee obtaining a stable product.

The technological complexity lies in the combination of two drugs with different rheological and solubility characteristics, on the one hand, we have a drug sensitive to humidity and light and on the other we have a fat-soluble drug, with poor flow and excessive adhesiveness (etoricoxib), which could cause interference in the stability, absorption and release mechanism.

The combination of both drugs will allow a better analgesia at reduced doses and a reduction in adverse effects.

### Example 3 Stability tests

Stability studies were carried out in accordance with current regulations on three batches of the described pharmaceutical composition. The results at 6 months in the condition of 40 °C and 75% relative humidity are shown below (Table 3):

**Table 3. Tramadol 50 mg / etoricoxib 90 mg**

| Determination | Specification | Result | | |
|---|---|---|---|---|
| | | Batch 1 | Batch 2 | Batch 3 |
| Tramadol hydrochloride content | 90.0% to 110.0% 50.0 mg/Over | 102.9% | 99.3% | 99.0% |
| Etoricoxib content | 90.0% to 110.0% 90.0 mg/Over | 100.2% | 99.9% | 99.4% |
| Tramadol hydrochloride dissolution | Q = 80.0% in 30 minutes | 100.8% | 101.4% | 104.8% |
| Etoricoxib dissolution | Q = 70.0% in 30 minutes | 103.6% | 103.6% | 105.54% |
| Organic impurities of Tramadol HCl | No more than 1.0% of total impurities | 0.24% | 0.23% | 0.19% |
| Organic impurities of etoricoxib | No more than 1.0% of total impurities | 0.06% | 0.06% | 0.06% |

### Example 4. Comparative Bioavailability Testing

The results obtained in a comparative bioavailability study of the combination etoricoxib with tramadol, allowed the inventors to conclude the adequate pharmacokinetic profile of the product when compared to the administration of the monodrugs alone. On the other hand, they allowed the inventors to guarantee the quality of the product formulation in combination of etoricoxib with tramadol and its excipients. Furthermore, the results obtained allowed the inventors to conclude that there is no interaction in the bioavailability of the formulations administered in combination. Additionally, the satisfactory compliance with the established quality standards, the current NOM-177-SSA1, the procedures, the protocols, the GLP and the Good Documentation Practices were verified, as support for the veracity of the study results.

For the pharmacokinetic and statistical analysis, data were received from 42 study subjects with a randomized crossover design, for three treatments, six sequences and three periods (3 x 6 x 3). Plasma concentration data were obtained each time, by medication. An analysis of variance (ANADEVA) was carried out for the parameters: Cₘₐₓ and ABC₀₋₇₂. In addition to descriptive statistics, the following tests were carried out: Classical Confidence Intervals, 90% (95%), and the *Schuirmann* Test*.* The calculations were carried out through the WINNONLIN^{®} version 8.0 program.

The statistical analysis to establish the non-pharmacokinetic interaction of the combination of etoricoxib + tramadol (equivalence in bioavailability) was carried out with the data of 38 subjects who completed the study. 42 subjects participated, who received three treatments corresponding to a single oral dose: Reference Drug "A" (90 mg of etoricoxib, Reference Drug "B" (50 mg of tramadol and test Drug "C" (etoricoxib 90 mg/tramadol 50 mg, Laboratorios Silanes, S.A. de C.V.), which were administered in three periods (separated by a 14-day washout period) and six sequences. The total dose administered was: etoricoxib 90 mg and tramadol 50 mg, see Table 4.

**Table 4. Assessed medicaments**

| | Medicament A (Reference | Medicament B (Reference) | Medicament C (Test) |
|---|---|---|---|
| International Common Denomination (ICD) | Etoricoxib | Tramadol | Etoricoxib/tramadol |
| Generic Denomination | Etoricoxib | Tramadol | Etoricoxib/tramadol |
| Pharmaceutical form | Tablet | Capsule | Envelope with granulate to be diluted in 100 mL of water |
| Formula | Each tablet contains 90 mg etoricoxib | Each capsule contains 50 mg | Each envelope contains etoricoxib 90 mg/tramadol 50 mg |
| Administered dosage | one 90 mg tablet | one 50 mg capsule | 100 mL equivalents to 90 mg of etoricoxib/50 mg tramadol |
| Batch | S018131 | 1902022 | 20A0811G1 |
| Expiration Date | March 2021 | January 2021 | January 2022 |

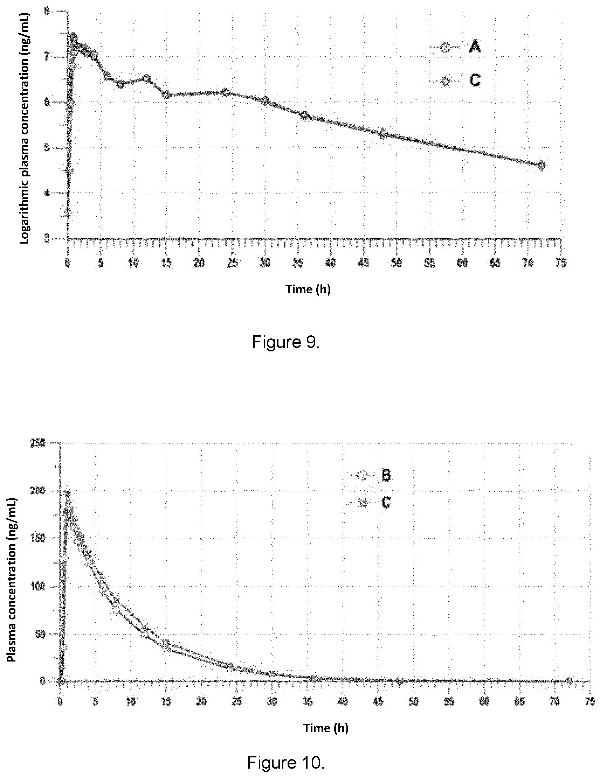

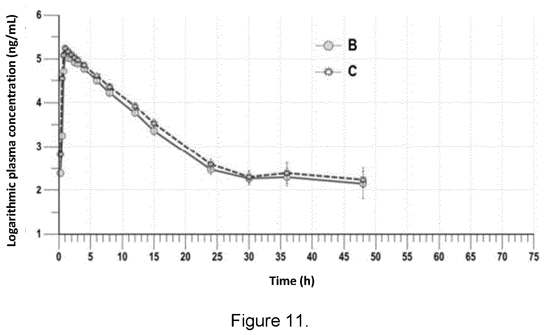

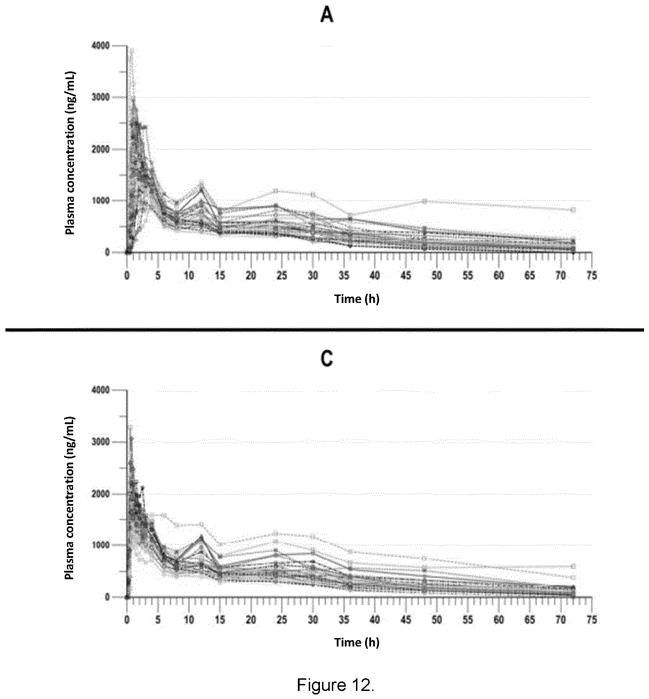

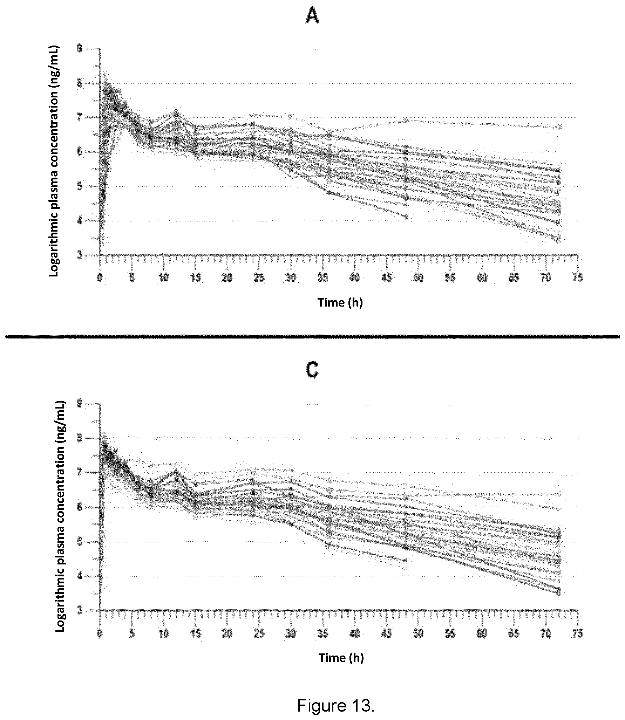

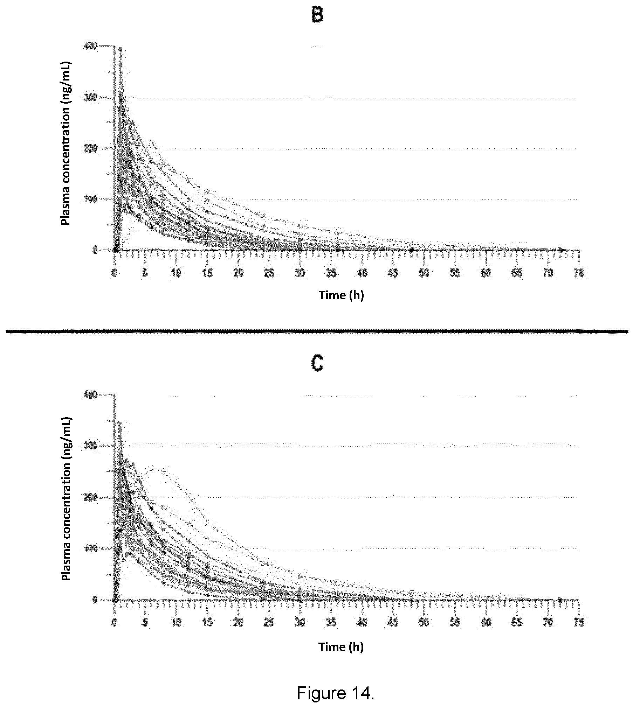

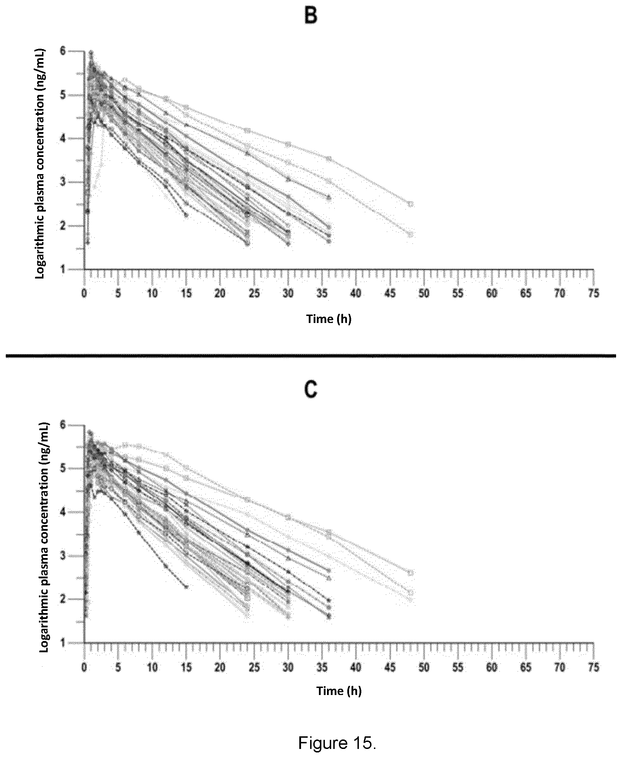

In order to visualize the possible differences between the formulations and/or treatments, the plasma concentration profiles (± standard error) were graphed with regards to time obtained in the participating subjects (see Figures 8-15).

The statistics to establish the equivalence of pharmacokinetic bioavailability must evaluate the difference between the treatments under study, through the logarithmic comparison of the pharmacokinetic parameters: Cₘₐₓ and ABC₀₋₇₂.

### a) Confidence intervals.

The statistical analysis was carried out on the equivalence of bioavailability of the drugs of interest based on the construction of confidence intervals, which conclude in favor of the equivalence of bioavailability (no pharmacokinetic interaction) if the calculated limits are included in the pre-established interval from 80 to 125, the above with logarithmically transformed data. These were analyzed with a confidence level of 90% (95%) and considering that the power of the statistical test should be greater than 0.80.

### b) Limit tests.

Limit tests are based on the rejection of the null hypothesis of non-equivalence to conclude with an alpha level of significance (a = 0.05) and a 90% confidence level, that the analytes present pharmacokinetic equivalence. The limit tests that were applied are the Schuirmann double-sided t test and the Anderson Hauck test on the ratio between the averages of the test and reference drugs, for the pharmacokinetic parameters of Cₘₐₓ and AUC₀₋₇₂.

Limit tests will conclude in favor of the bioavailability equivalence if the probability that the ratio is less than 80% and greater than 125% (less than 75% and greater than 133% in the case of high pharmacokinetic variability), is less than 0.05.

Table 5 below presents the results of the statistical tests applied to the pharmacokinetic parameters of Cₘₐₓ and AUC₀₋₇₂ of etoricoxib, obtained for the two assessed treatments (A vs C). The purpose is to evaluate the possible differences between the treatments, applying a confidence level of 90% (95%) and considering all the 38 subjects who completed the study.

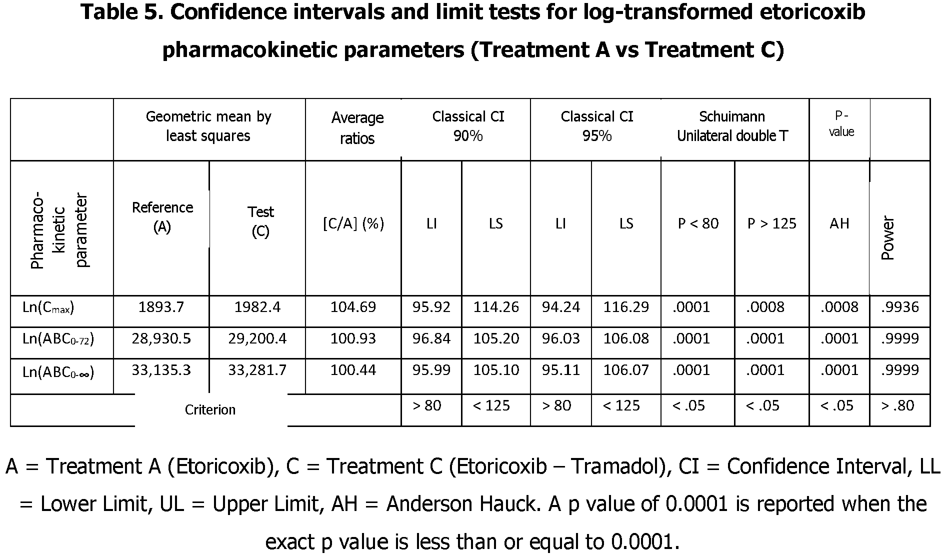

Table 6 below presents the results of the statistical tests applied to the pharmacokinetic parameters of Cₘₐₓ and AUC₀₋₇₂ of tramadol, obtained for the two assessed treatments (B vs C). The purpose is to evaluate the possible differences between the treatments, applying a confidence level of 90% (95%) and considering all the 38 subjects who completed the study.

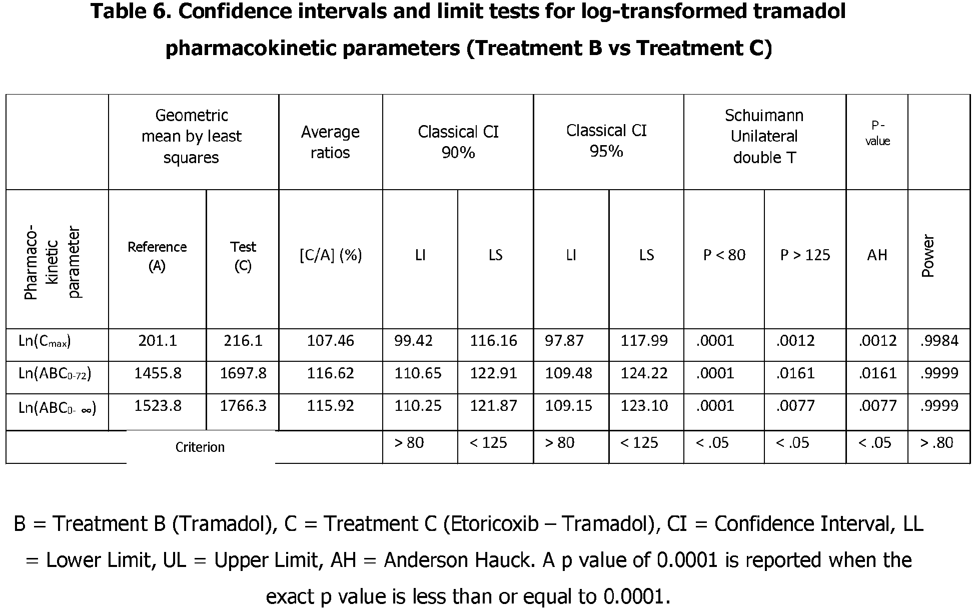

After administering a single oral dose corresponding to 90 mg of etoricoxib and 50 mg of tramadol in tablet and capsule dosage form, the reference drug product A, etoricoxib, 90 mg tablet, of reference drug product B, tramadol, 50 mg capsule, and test drug C, etoricoxib/tramadol in sachet dosage form with granules to be diluted in 100 mL of water, etoricoxib 90 mg/tramadol 50 mg, manufactured by Laboratorios Silanes, S.A. de C.V., batch 20A081G1, the pharmacokinetic parameters of Cₘₐₓ and area under the plasma concentration curve with respect to time (AUC₀₋₇₂) were determined.

The comparative analysis for pharmacokinetic parameters was performed using the classical and Schuirmann confidence interval approach. In the case of the classical confidence interval, it is a symmetrical interval around the difference in averages between the test and reference drug (CP-CR) and it is not symmetrical around zero, in the same way the classical confidence interval is symmetrical around the CP/CR ratio and not around unity.

The classical confidence intervals for etoricoxib (contrast A vs C) met the 80-125% criterion for log-transformed data, in each of the two pharmacokinetic variables, Ln (Cₘₐₓ) and Ln(AUC₀₋₇₂).

With regards to the pharmacokinetic parameter ABC₀₋₇₂, which is related to the degree or magnitude of absorption, in the classical confidence intervals, it is found that they are located within the range of 80-25% for logarithmically transformed data, the same case as the Schuirmann and Anderson Hauck tests **(5)** showing a low probability that the AUC₀₋₇₂ values of the test drug (etoricoxib/tramadol) are outside the 80-125% ("p" value less than 0.05).

The classical confidence intervals for tramadol (contrast B vs C) met the 80-125% criterion for log-transformed data, in the two pharmacokinetic variables, Ln (Cₘₐₓ) and Ln(AUC₀₋₇₂).

With regards to the pharmacokinetic parameter ABC₀₋₇₂, associated to tramadol, it is understood that the classic confidence intervals are within the range of 80-125% for logarithmically transformed data, the same case as the Schuirmann and Anderson Hauck tests (5) showing a low probability that the AUC₀₋₇₂ values of the test drug (etoricoxib/tramadol) are outside 80-125% (lower "p" value of 0.05).

All the criteria used for non-pharmacokinetic interaction were met, given that for each of the contrasts (C vs A and C vs B) where: A is etoricoxib monotherapy, reference drug, B is tramadol monotherapy, reference drug, C is a fixed-dose combination of etoricoxib/tramadol, test drug, in the variables Ln(Cₘₐₓ) and Ln(ABC₀₋₇₂):
(1) the lower limit of the classical interval (90% CI) is greater than 80 and the upper limit of the classical interval is less than 125,
(2) the classical 95% confidence interval does not present values outside the 80 - 125% interval,
(3) the p values associated with the Schuirmann test for P < 80 are less than 0.05 (p ≤ .0001),
(4) the p values associated with the Schuirmann test for P > 125 are less than 0.05 (p ≤ .0161),
(5) the Anderson Hauck test provides p values lower than .05, (p ≤ .02), so it is possible to reject the hypothesis of no pharmacokinetic interaction (α = 0.05).

Furthermore, it is noted that the powers were adequate, greater than .99 for the two variables under study, *Ln(Cmax)* and *Ln(ABC₀₋₇₂),* in both contrasts, see Tables 6 and 7.

Thus, in the pharmacokinetic profile of the fixed-dose combination of etoricoxib/tramadol and its concomitant dosing, it was observed that there are no statistically significant differences in the values of Cₘₐₓ, AUC ₀₋ₜ and AUC _{0-inf}, the time to reach the Cₘₐₓ for the fixed-dose combination was lower than etoricoxib as a single drug, while for tramadol, it was similar. The 90% confidence intervals (CI) observed for etoricoxib were 95.92-114.26, 96.84-105.20 and 95.99-105.10, respectively, and tramadol 90% CI; 99.42-116.16, 110.65-122.91 and 110.25-121.87, respectively. These results demonstrate that the reduction in the dose of tramadol (tramadol-sparing effect) is not related to pharmacokinetic differences of the fixed-dose formulation of etoricoxib/tramadol, but rather to a synergistic effect based on pharmacodynamics inherent to the mechanisms of action of the drugs when administered in combination and provides a multimodal therapeutic approach.

### Example 5. Dissolution tests

The dissolution test (*in vitro test*) serves to determine the rate (amount/time) and extent (total amount) at which a drug is released from the dosage form; in the case of the dissolution profile, it corresponds to the quantification at different times of the dissolved drug under standardized conditions. The importance of the dissolution test lies in the following:
a) It is a guide for the development of new formulations during product development: it allows evaluating the possible interference of excipients or the manufacturing process on the release of the drug.
b) Process control and quality assurance helps ensure the continued quality of the product and its optimization after a change in manufacturing, formulation, manufacturing site and process escalation.
c) Indicator of *in vivo* development: it is an indicator of bioavailability, it allows establishing the correlation between *in vitro* parameters with bioavailability results.

Dissolution profile testing was performed for granulated tramadol hydrochloride/etoricoxib to reconstitute 50/90 mg.

The quantification of etoricoxib in the study was carried out through a method previously validated under the criteria of the Mexican Official Standard NOM-177-SSAA-2013, the method was carried out using high-resolution liquid chromatography taking samples at 20, 40, 60, and 80 min. The dissolution medium used consisted of a 0.1 N hydrochloric acid solution.

The method for the quantification of tramadol hydrochloride was similarly validated under the criteria of the Mexican Official Standard NOM-177-SSAA-2013, the method was carried out using high-resolution liquid chromatography taking samples at 20, 40, 60, and 80 min. The dissolution medium used consisted of a 0.1 N hydrochloric acid solution.

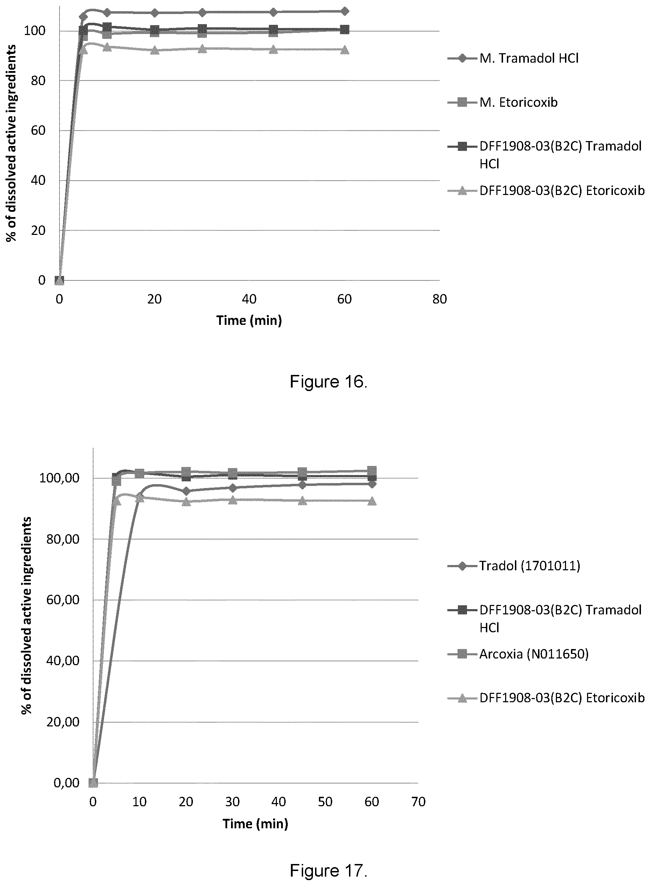

The percentage of tramadol hydrochloride and etoricoxib in their respective dissolution medium for both the reference drug and the test drug was greater than 85% within the first 20 min, reason for which these results show that the evaluated products can be accepted as equivalent (see Figures 16 and 17).

### Example 6. Clinical tests of efficacy and safety of the fixed-dose combination of etoricoxib - tramadol for the management of acute pain and its synergy

Within the available literature, a pre-clinical study evaluated possible interactions (additive or potentiating) in the antinociceptive effects of etoricoxib and tramadol in combination against mechanical hyperalgesia induced by spinal cord injury in rats. The authors concluded that the synergistic antihyperalgesic action of this combination has utility in mechanical hyperalgesia associated with spinal injury that could be extrapolated to clinical application.

Different fixed-dose combinations have been evaluated in the treatment of pain, including tramadol/paracetamol, tramadol/diclofenac, codeine/ibuprofen, codeine/paracetamol, oxycodone/paracetamol and tramadol/dexketoprofen, either in comparison with the monodrug or in relation to any of these combinations. Like etoricoxib/tramadol, other combinations of NSAIDs with tramadol versus paracetamol/tramadol have been evaluated, showing similar results in the treatment of pain.

In 2006, Chandenwale et al, evaluated the analgesic efficacy of fixed-dose combinations of diclofenac/tramadol versus paracetamol/tramadol in patients with acute musculoskeletal conditions, post-operatory pain after orthopedic surgery, osteoarthritis and rheumatoid arthritis, identifying a greater reduction in pain intensity and lower frequency of adverse events with the diclofenac combination after 5 days of treatment. This study presented superior results with the fixed dose combination of Diclofenac/Tramadol using a schedule with a lower dose (1 tablet twice a day vs 2 tablets every 4-6 hours without exceeding 8 tablets), greater adherence and a lower number of adverse events.

Currently, the use of Paracetamol 325 mg/Tramadol 37.5 mg is recommended for the treatment of lower back pain, based on a minimum dosage regimen of 3 doses and a maximum of 10 doses in 24 hours, the equivalent of 975 mg to 3250 mg of paracetamol and 112.5 mg to 375 mg of Tramadol per day, for a period of up to 4 weeks, this treatment is one of the most used, although it is not necessarily the safest analgesic.

The combination of Etoricoxib/Tramadol provides effective pain relief through multimodal management due to the different mechanisms of action of its components, achieving a faster reduction in pain intensity compared to conventional therapy. The multimodal approach provided by this combination not only improves treatment adherence, but also achieves a tramadol-sparing effect (pain control with lower doses of tramadol and, therefore, lower incidence of adverse events related to it). For the relief of acute pain of moderate to severe intensity, multimodal analgesic regimens will be preferred over monotherapy and, even more so, management with tramadol, through timely interventions, to provide an immediate pain relief and prevent the development of complications associated with the same. One of the most used models to evaluate the development of pharmacological products with analgesic therapeutic potential is that of lower back pain, where the therapeutic effects of new analgesic agents can be studied.

The definition of a clinically significant change in pain intensity is undoubtedly highly variable from study to study. Therefore, a study was conducted to evaluate the efficacy and safety of the fixed-dose combination of etoricoxib 90 mg and tramadol 50 mg compared to the fixed-dose therapy of paracetamol 325 mg and tramadol 37.5 mg for the management of acute lower back pain.

In the "Confirmatory study of efficacy and safety of the fixed-dose combination of Etoricoxib/Tramadol versus Paracetamol/Tramadol for the management of acute lower back pain conducted by Laboratorios Silanes S.A de C.V., the drug under investigation (Etoricoxib 90 mg/Tramadol 50 mg ) is indicated as a single dosage regimen every 24 hours, obtaining the same therapeutic effect as the Paracetamol/Tramadol 325 mg/37.5 mg combination (administered every 8 hours for a final dose of 975 mg/112.5 mg, respectively).

The study design was a phase IIIb, longitudinal, multicenter, randomized, open-label, clinical trial to evaluate the efficacy and safety of the fixed-dose combination of etoricoxib 90 mg and tramadol 50 mg compared to the fixed-dose therapy of paracetamol 325 mg and tramadol 37.5 mg for the management of acute lower back pain in 62 adult patients per treatment group (for a total of 124 patients).

The inclusion criteria included:
1. Any gender.
2. Agree to participate in the study and give their written informed consent.
3. Age >18 years old and ≤60 years old at the beginning of the study.
4. Diagnosis of acute lower back pain as a first-time episode or after another episode maximum 6 months before and with a duration of no more than 6 weeks.
5. Patient with lower back pain reported as moderate to severe intensity (Visual Analog Scale of Pain VAS > 4 cm).
6. Women of childbearing age who have an acceptable contraceptive method (e.g. barrier, oral hormonal, injectable, subdermal).

Table 7 shows the test and reference products.

**Table 7. Test and reference products**

| | |
|---|---|
| Test Product: | A. etoricoxib/tramadol |
| | Orally administered, a granulated etoricoxib/tramadol envelope diluted into 100 mL of water, every 24 horas, per 7 days. |
| | Concentration: 90 mg/50 mg |
| | Manufacturer: Laboratorios Silanes S.A. de C.V. |
| | Batch: 20A081G1 |
| Reference Therapy | B. paracetamol/tramadol |
| | Orally administered, a tablet, every 8 hours, per 7 days. |
| | Concentration: 324 mg/37.5 mg |

For the evaluation and effectiveness criteria, the following parameters were followed:

### Effectiveness

### Primary variables:

- Average change in pain reported through the VAS at 3, 5 and 7 days with respect to its baseline measurement by treatment group.

### Secondary variables:

- A proportion of patients achieving a 30% reduction in pain intensity at 7 days by treatment group.
- Degree of disability due to lower back pain measured using the Oswestry Disability Scale at baseline and after 7 days, by treatment group.
- Degree of inability to perform daily activities using the Roland-Morris scale, baseline and after 7 days by treatment group.

### Safety

- Frequency and intensity of adverse events occurring during the study between treatment groups.

The efficacy results showed the overall non-inferiority of etoricoxib-tramadol in terms of effectiveness and safety for the management of acute lower back pain after 7 days of treatment. The data obtained from the patients included in the present study confirm the effectiveness of the combination treatment based on etoricoxib 90 mg/tramadol 50 mg in the management of acute lower back pain during a 7-day follow-up, observing a constant reduction in intensity of pain and an improvement in the degree of disability due to lower back pain and to perform daily activities.

By analyzing the data at each follow-up time, it was shown that the test product based on etoricoxib/tramadol is superior to the standard Paracetamol/Tramadol therapy since an analgesic therapeutic effect is achieved in earlier stages and the proportion of patients with a decrease in pain score is greater. In the fixed-dose combination group of etoricoxib/tramadol, a greater reduction in pain could be observed within the first days of treatment (3 and 5 days) compared to paracetamol/tramadol therapy, hence the therapeutic effects of the test product are obtained earlier, which represents an advantage over the comparison group. The proportion of patients who achieved a change in the degree of pain intensity, from the initial categories of severe and moderate to mild and no pain at 3 and 5 days, was also higher in the etoricoxib/tramadol group, once more it is demonstrated that the test product is superior to the reference product. In addition, a significantly higher proportion of patients with a response to treatment was reported, considering this as a decrease in pain intensity of at least 30% on the VAS, this reported on the third day of follow-up.

This is because the test product in the fixed-dose combination of etoricoxib/tramadol has been formulated in a granular pharmaceutical form, which facilitates absorption in the gastrointestinal tract and, accordingly, early blood concentrations that translate into a anticipated analgesic effect. For its part, the reference drug based on paracetamol/tramadol in pharmaceutical tablet form must go through a disintegration process at the gastrointestinal level, which delays its absorption and results in a delayed analgesic effect.

On the other hand, within each treatment group, significant changes were identified in the median pain on the third, fifth and seventh day, with respect to their baseline measurement (p=0.001). By obtaining the magnitude of change in pain assessments on days 3, 5, and 7 with respect to baseline, differences were observed between the treatment groups at 3 days (p=0.054) and 5 days (p=0.041). When evaluating the proportion of patients with a response to treatment (30% (3 cm) reduction in VAS), the results show statistically significant differences when comparing the treatment groups on the third day of follow-up [p= 0.008, 95% CI 0.241 (0.061- 0.421)] equaling its effectiveness at 5 and 7 days. This is why, although at the final follow-up on day 7 there is non-inferiority between the treatments administered, the test drug etoricoxib/tramadol is capable of providing earlier and superior analgesic effects from the third day of follow-up.

In addition to the VAS, the improvement in the inability due to lower back pain to perform daily activities (Oswestry and Roland-Morris) was evaluated in the group of patients who received etoricoxib/tramadol, especially the improvement in the inability to perform daily activities (p =0.068). According to the results of the Oswestry and Roland-Morris scales, the etoricoxib/tramadol group reports a higher proportion of patients with improvement in disability compared to the paracetamol/tramadol group, reporting a change in the grade classification from severe to moderate and minimal disability.

Finally, it is important to highlight that, due to the pharmaceutical form (granules) and its pharmacokinetics that allows administration every 24 hours (once a day) to the dosage of etoricoxib/tramadol, compared to the paracetamol/tramadol group that warrants administration every 8 hours (3 times a day), greater adherence was observed for the group treated with the etoricoxib/tramadol test drug compared to the treatment with paracetamol/tramadol.

The above allowed the inventors to identify the clinical benefits and the superiority of the etoricoxib/tramadol combination, such as greater rate in achieving a therapeutic response, an early analgesic response, pain reduction similar to paracetamol/tramadol after 7 days of treatment, use of lower doses of tramadol per day (opioid-sparing effect); which translates into lower risks of dependence, decreased use of opiates and better adherence to treatment.

Regarding the safety of the products under investigation, no relevant data that had not been previously reported on the products used in monotherapy were found. Among all adverse events reported in the Silanes study, the Etoricoxib/Tramadol group reported a lower proportion of AEs compared to the group receiving Paracetamol/Tramadol. In the safety results, for example, the information of 124 patients was evaluated, of which 38 of them presented at least one adverse event (AE), 16 subjects in the etoricoxib/tramadol group and 22 in Paracetamol/tramadol, reporting a total of 79 AEs (38 events (48.1%) with the test drug and 41 events (51.9%) with the comparator). In relation to severity, all events were classified as non-serious (100%). No deaths were reported in the study. According to the severity of the event, a greater proportion of AEs classified as "mild" compared to "moderate" was observed in both treatments (73.4% vs 26.6%). The paracetamol/tramadol group reported adverse events with greater severity, these being moderate compared to etoricoxib/tramadol (41.5% vs 10.5%), this difference was considered statistically significant (p=0.002). The most affected SOCs were gastrointestinal disorders and nervous system disorders, with 33 (41.7%) events [19 (57.5%) of them in the etoricoxib/tramadol group and 14 (42.5%) in the Paracetamol/tramadol group] and 32 (40.5%) events [14 (43.7%) of them in the etoricoxib/tramadol group and 18 (56.3%) in the Paracetamol/tramadol group], respectively. The most frequent events were nausea (17.7%) and dizziness (16.4%). No clinically significant differences were observed in biochemical markers and vital signs. All of the above allows us to conclude that at the time of carrying out the evaluation no safety or risk signal was identified that could modify the benefit-risk balance of the patients with the use of the study drugs, reason for which the safety profile remains favorable according to the information described.

In this way, it was possible to identify that a greater proportion of patients in the Etoricoxib/Tramadol group reported a reduction in pain intensity ≥30% in a more acute period (during the first 3 days) compared to the Paracetamol/Tramadol group. These data coincide with those reported by Meloncelli, et al in a study that compared the analgesic efficacy and tolerability of the combination Tramadol/Dexketoprofen 75 mg/25 mg versus diclofenac/thiocolchicoside 75 mg/4 mg in patients with acute lower back pain. Furthermore, they observed that the highest proportion of subjects who responded (defined as a reduction in pain intensity of at least 30%) to the treatment corresponded to the group with the tramadol/dexketoprofen combination.

In conclusion, the data obtained from the patients included in the present study confirm the efficacy and safety of the combination of etoricoxib/tramadol for the treatment of acute lower back pain during a 7-day follow-up, observing a constant reduction in the intensity of the pain (VAS) and an improvement in the degree of disability due to lower back pain (Oswestry) and to perform daily activities (Roland-Morris).

It is important to note that, due to the pharmaceutical form (granules) and the dosage of etoricoxib/tramadol, a higher percentage of adherence was observed compared to the Paracetamol/tramadol treatment group (98.3 [CI 95%, 96.0-100] vs 94.5 [CI 95%, 90.5-98.5]).

By comparing the doses used, it can be demonstrated that both treatments administered have comparable therapeutic efficacy, although the treatment based on Etoricoxib/Tramadol is more potent from a pharmacological point of view since the desired analgesic therapeutic effects are achieved with lower doses (90 mg/50 mg vs 975 mg/ 112.5 mg). The results obtained through the clinical study demonstrate an analgesic synergy in patients affected by acute lower back pain, in addition to a tramadol-sparing effect. The above results show the clinical benefits of Etoricoxib/Tramadol in a convenient dosing schedule, such as faster therapeutic response, therapeutic equivalence at the end of the evaluation period, and tramadol-sparing effect (66.6% reduction in tramadol dose per day), which translates to: decreased risk of dependence, incidence of adverse events dependent on the tramadol dose (such as nausea, vomiting, drowsiness, etc.), reduced pill burden which results in a better therapeutic adherence.

At the time of carrying out the evaluation no safety or risk signal was identified that could modify the benefit-risk balance of the patients with the use of the study drugs, reason for which the safety profile remains favorable according to the information above described.

By decreasing the dose of tramadol per day, a lower number of patients with adverse events related to this product were observed in the etoricoxib-tramadol combination.

### Advantages of the invention and industrial application

One of the main problems in those medicaments with synergistic compositions of known drugs and of known concentration is the difficulty in ensuring that their stability, dissolution and bioavailability are maintained, after improving their organoleptic properties and once administered to the patient; especially when the drugs are highly sensitive to light and humidity.

In this sense, even and despite being within the regulatory parameters of dissolution and bioavailability, the results frequently tend towards data close to the lower limit of the specification, which causes the therapeutic effect to take time to occur or a higher dose to be required.

In the prior art, an attempt has been made to solve the problem of combining both drugs for the treatment and control of pain and inflammation. The documents talk a lot about bilayers and independent systems. There were even those who approached the problem from a new crystalline form. The only way they approach combining tramadol and etoricoxib is that there is no actual physical interaction of the APIS, otherwise they are not combined. Likewise, there is a need to improve the synergistic effect, increase bioavailability, improve dissolution, stability and at the same time ensure a decrease in side effects once administered to the patient. The lack of solutions and alternatives to the above causes the therapeutic effect to take time to occur or a higher dose to be required. Furthermore, the patents and scientific contributions described above do not show interest in resolving the impact on the organoleptic properties of the drug, which affect its stability, dissolution and bioavailability.

On the other hand, in addition to the problems of side effects due to the conformation of high doses of tramadol and etoricoxib, there is the technical problem of combining a drug with high sensitivity to light and humidity, with another fat-soluble drug with poor flow and excessive adhesiveness since there could be interference in the stability, absorption and release mechanism.

The present invention relates to a pharmaceutical composition of a combination of an opioid analgesic drug such as tramadol with an NSAID such as etoricoxib, for the manufacture of a medication useful in the treatment and control of pain such as neuropathic pain and/or chronic and acute pain, in a single monophasic, stable, immediate-release system, with a synergistic effect and which maintains dissolution and bioavailability in the field of treatment and control of pain and inflammation, since the synergistic effect of the drugs with low doses while maintaining the dissolution and bioavailability of the composition, without losing the therapeutic effect once administered, as demonstrated with regards to the pharmacokinetic bioavailability after having administered the composition of the present invention and the reference oral formulations.

On the other hand, in the case of a combination of active ingredients, it may be sufficient to demonstrate that the effect of the combination is greater than that obtained with each of the active ingredients used separately. This combined technical effect can also be based on more indirect aspects, such as the reduction of harmful side effects, the optimization of the pharmacokinetics or the bioavailability of at least one of the active ingredients, the possibility of administering the medicament by a different route, the improvement of patient compliance with treatment, reducing dose frequency, etc. This technical effect (whether a synergy or any other advantage) is not suggested by the prior art.

The enhanced analgesic effect of the present invention is due to the combination of two pharmacodynamic mechanisms that complement each other thanks to the combination of an NSAID (etoricoxib) plus an opioid (tramadol). Additionally, this combination represents a safety profile superior to the treatment with monodrugs since it allows the adverse events derived from the use of opioids to be reduced because when combining etoricoxib with tramadol, therapeutic results are obtained with a daily dose of the product. Furthermore, an improved stability is presented regarding the pharmaceutical profile.

Currently, the use of paracetamol 325 mg/tramadol 37.5 mg is recommended for the treatment of lower back pain, prescribed in a dosage scheme of a minimum of 3 doses and a maximum of 10 doses in 24 hours, the equivalent of 975 mg to 3250 mg of paracetamol and 112.5 mg to 375 mg of Tramadol per day, for a period of up to 4 weeks [Dhillon, S., Tramadol/paracetamol fixed-dose combination. Clinical drug investigation, 2010. 30(10): p. 711-738].

However, the combination of Etoricoxib/Tramadol provides effective pain relief through multimodal management due to the different mechanisms of action of its components, achieving a faster reduction in pain intensity compared to conventional therapy. The multimodal approach provided by this combination not only improves treatment adherence, but also achieves a tramadol-sparing effect (pain control with lower doses of tramadol and, therefore, lower incidence of adverse events related to it). For the relief of acute pain of moderate to severe intensity, multimodal analgesic regimens will be preferred over monotherapy and, even more so, management with tramadol, through timely interventions, to provide an immediate pain relief and prevent the development of complications associated with the same. In this way, and in comparison, the product under investigation (etoricoxib 90 mg/tramadol 50 mg) is indicated in a dosing schedule of a single dose every 24 hours, obtaining the same therapeutic effect. The data obtained demonstrate that this analgesic synergy turns out to be more powerful (same therapeutic effect obtained with a lower dose) than the combination of paracetamol 325 mg / tramadol 37.5 mg. For example, it was possible to identify that a greater proportion of patients in the Etoricoxib/Tramadol group reported a reduction in pain intensity ≥30% in a more acute period (during the first 3 days) compared to the Paracetamol/Tramadol group. Furthermore, among all adverse events reported in the Silanes study, the Etoricoxib/Tramadol group reported a lower proportion of AEs compared to the group receiving Paracetamol/Tramadol.

Moreover, the pharmacokinetic profile of the etoricoxib/tramadol combination of the present invention demonstrates that the reduction in the dose of tramadol (tramadol-sparing effect) is not related to pharmacokinetic differences of the fixed-dose formulation of etoricoxib/tramadol, but rather to a synergistic effect based on pharmacodynamics inherent to the mechanisms of action of the drugs when administered in combination and provides a multimodal therapeutic approach.

The previous results show the clinical benefits of the etoricoxib/tramadol combination, such as obtaining a therapeutic response in a shorter period of time, therapeutic equivalence at the end of the evaluation period, use of lower doses of tramadol per day (reduction of 66.6% per day), which translates into: decreased risk of dependence, decreased incidence of dose-dependent adverse events of tramadol and better therapeutic adherence. Therefore, no warning signs or risks were identified that could modify the risk-benefit balance with the use of the etoricoxib/tramadol combination of the present invention.

## Claims

1. A pharmaceutical composition characterized because it comprises tramadol in an effective amount of 50 mg and etoricoxib in an effective amount of 90 mg, wherein the composition is a granule for reconstitution.

2. The pharmaceutical composition according to claim 1, further characterized because it comprises at least one of a binder, a disintegrant, a sweetener, a flavoring and a diluent.

3. The pharmaceutical composition according to claim 2, further characterized because it comprises a diluent, a binder and two sweeteners.

4. The pharmaceutical composition according to claim 3, further characterized because it comprises:
- tramadol hydrochloride in an effective amount of 50 mg;
- etoricoxib in an effective amount of 90 mg;
- isomalt 801 as diluent in an amount of 36% by weight of the composition;
- polyethylene glycol as a binder in an amount of 43.20% by weight of the composition;
- croscarmellose sodium as disintegrant in an amount of 5.0% by weight of the composition;
- acesulfame potassium as the first sweetener in an amount of 3.0% by weight of the composition;
- sucralose as a second sweetener in an amount of 2.0% by weight of the composition; and
- mint-peppermint flavor in an amount of 8.0% by weight of the composition.

5. A process for the manufacture of a pharmaceutical composition according to any one of claims 1 to 4, characterized because it comprises the following steps:
a) mixing the active ingredients (tramadol and etoricoxib) with pharmaceutically acceptable excipients (polyethylene glycol, isomalt 801, croscarmellose sodium, acesulfame potassium and sucralose) previously screened for 5 minutes (mixture 1);
b) granulate mixture 1 for 20-30 minutes until it reaches a temperature between 54-55.2 °C;
c) cooling the granules to a temperature of 25 to 28 °C;
d) screening the granules from step d), thus obtaining a screen 3;
e) mixing screen 3 with the mint-peppermint flavor for 5 minutes.

6. The process according to claim 5, further characterized because the granulations are carried out by a fusion granulation process.

7. A pharmaceutical composition as described in any one of claims 1 to 4 for use in the treatment of pain and inflammation.

8. The pharmaceutical composition for use according to claim 7, wherein the pain is selected from the group consisting of neuropathic pain, chronic pain and acute pain.

9. The pharmaceutical composition for use according to claims 7 or 8, wherein the composition is adapted to be administrable every 24 hours.
